# FASCICULE DE BREVET EUROPEEN

(11) **EP 1 397 111 B1**
(45) Date de publication et mention de la délivrance du brevet: **20.09.2006**
(21) Numéro de dépôt: 02738293.6
(22) Date de dépôt: 31.05.2002
(51) Int. Cl.: A61K 8/19, A61K 8/41, A61Q 5/00, A61Q 19/00

(54) **COMPOSITION COSMETIQUE COMPRENANT DES PARTICULES DE CARBONATE DE CALCIUM ET UNE ASSOCIATION DE TENSIOACTIFS**
KOSMETISCHE, CALCIUMCARBONATPARTIKEL UND EINE KOMBINATION VON TENSIDEN ENTHALTENDE ZUSAMMENSETZUNG
COSMETIC COMPOSITION COMPRISING CALCIUM CARBONATE PARTICLES AND A COMBINATION OF SURFACTANTS

(30) Priorité: 31.05.2001 FR 0107162
(43) Date de publication de la demande: 17.03.2004
(73) Titulaire: L'ORÉAL, 75008 Paris (FR)
(72) Inventeur: PERRON, Béatrice, F-78350 Jouy en Josas (FR); RESTLE, Serge, F-95390 Saint-Prix (FR); GIROUD, Franck, F-92110 Clichy (FR); SAMAIN, Henri, F-91570 Bièvres (FR)
(74) Mandataire: Le Blainvaux Bellegarde, Françoise
(86) Numéro de dépôt international: PCT/FR2002/001845
(87) Numéro de publication internationale: WO 2002/096377

(56) Documents cités:
- WO-A-99/25312
- FR-A- 2 229 389
- US-A- 4 895 722

## Description

La présente invention est relative à une composition cosmétique, notamment capillaire, contenant, dans un milieu cosmétiquement acceptable, des particules de carbonate de calcium et une association de tensioactifs. Elle vise également un procédé de traitement cosmétique des cheveux comprenant l'application de cette composition ainsi que son utilisation en tant que produit capillaire rincé.

Pour le nettoyage et/ou le lavage des cheveux, l'utilisation de compositions capillaires détergentes (ou shampooings), à base essentiellement d'agents tensioactifs classiques de types notamment anionique, non-ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage et une douceur nettement accrue par rapport à ce qui peut être obtenu avec les compositions nettoyantes correspondantes qui en sont exemptes.

Cependant, ces avantages cosmétiques s'accompagnent malheureusement également, sur cheveux séchés, de certains effets cosmétiques jugés indésirables, à savoir un alourdissement de la coiffure, un manque de lissage.

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion entre les fibres affectant le coiffage. Ces inconvénients sont accentués dans le cas de cheveux fins, qui manquent de nervosité et de volume.

Il a déjà été proposé l'utilisation de particules dans des compositions rincées, de façon à améliorer le toucher et l'aspect des cheveux. A titre d'illustration, le brevet US 5 334 376 propose l'ajout de particules de carbonate de calcium dans des compositions de conditionnement des cheveux contenant une silicone, un alcool gras et une amide.

Dans la demande de brevet DE 199 46 784, il a également été proposé l'utilisation de particules de différents oxydes, hydroxydes, carbonates, silicates ou phosphates, dans des compositions capillaires, pour réduire l'aspect gras des cheveux. Il est prévu, en toutes généralités, d'associer ces particules aux ingrédients classiques des shampooings.

Toutefois, et malgré les progrès réalisés récemment dans le domaine des produits capillaires rincés et notamment des shampooings, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques, de meilleures performances.

La Demanderesse a découvert, de manière surprenante et inattendue, qu'en choisissant judicieusement la base de tensioactifs et l'agent de conditionnement, associés avec des particules de carbonate de calcium, il était possible d'améliorer les résultats obtenus avec les produits cosmétiques, notamment capillaires rincés, en terme de propriétés cosmétiques et de mise en forme. En particulier, on apporte de la texture aux cheveux (sensation d'épaisseur accrue) et une meilleure tenue de la coiffure.

L'invention a pour objet une composition cosmétique, notamment capillaire, comprenant, dans un milieu cosmétiquement acceptable :
(a) des particules solides contenant au moins 10 % en poids de carbonate de calcium ;
(b) une association d'au moins deux tensioactifs, choisie parmi les associations:
   - d'au moins un tensioactif anionique (i) et d'au moins un tensioactif amphotère (ii) ou
   - d'au moins un tensioactif anionique (i) et d'au moins un tensioactif non ionique (iii) ;
(c) un polymère cationique dont la densité de charge cationique est inférieure ou égale à 7 meq/g et, de préférence, supérieure ou égale à 0,05 meq/g.

De préférence, le rapport pondéral de concentrations du ou des tensioactifs anioniques aux tensioactifs non ioniques et/ou amphotères est supérieur à 1.

Un autre objet de la présente invention consiste en un procédé de traitement cosmétique des cheveux mettant en oeuvre la composition selon l'invention.

L'invention a encore pour objet l'utilisation de la composition en cosmétique capillaire, notamment en application capillaire rincée.

D'autres objets, caractéristiques, aspects et avantages de l'invention apparaîtront encore plus clairement à la lecture de la description et des divers exemples qui suivent.

### PARTICULES DE CARBONATE DE CALCIUM

Les particules contenant au moins 10 % en poids de carbonate de calcium présentent; de préférence, une taille primaire moyenne en nombre comprise entre 2 nm et 2 µm, plus préférentiellement entre 5 et 500 nm, et plus préférentiellement encore entre 10 et 250 nm.

Les particules selon l'invention peuvent, par exemple, avoir une forme quelconque, par exemple la forme de sphères, de paillettes, d'aiguilles, de plaquettes ou des formes totalement aléatoires.

Conformément à la présente invention, la particule peut être une particule massique formée entièrement de carbonate de calcium. Le carbonate de calcium peut également constituer totalement ou partiellement le coeur de la particule, ce dernier étant recouvert d'un autre constituant, comme par exemple, un oxyde, un silicate ou un métal. Le carbonate de calcium peut encore former uniquement le revêtement d'un substrat de constitution chimique différente, comme par exemple un oxyde, un silicate ou un métal.

Au sens de la présente invention, on entend par *"taille primaire de particule",* la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Dans le cas où les particules sont formées par du carbonate de calcium et d'autres charges, le carbonate de calcium se trouve à l'état libre et ne forme pas de liaisons chimiques avec les autres charges. Il s'agit alors d'un alliage entre le carbonate de calcium et d'autres charges, notamment avec des oxydes de métaux ou de métalloïdes, en particulier obtenu par fusion thermique de ces différents constituants.

Lorsque les particules contenant au moins 10 % en poids de carbonate de calcium comprennent, en outre, un oxyde de métal ou de métalloïde, celui-ci est notamment choisi parmi l'oxyde de silicium, de bore ou d'aluminium.

De préférence, les particules contiennent au moins 50 % en poids de carbonate de calcium, mieux encore au moins 70 % en poids, et les particules constituées à plus de 90 % en poids de carbonate de calcium sont particulièrement préférées selon la présente invention.

Plus avantageusement encore, les particules contenant au moins 10 % en poids de carbonate de calcium, sont des particules de carbonate de calcium substantiellement pur.

Les particules contenant du carbonate de calcium selon l'invention sont, notamment, utilisées en une quantité comprise entre 0,01 % et 30 % en poids, et de préférence entre 0,05 % et 5 % en poids par rapport au poids total de la composition.

Le carbonate de calcium convenant dans les compositions de la présente invention peut être d'origine naturelle ou peut être d'origine synthétique. Dans ce dernier cas, il peut être obtenu à partir d'oxyde de calcium, de peroxyde de calcium, d'acétate ou d'éthylate de calcium.

La composition selon l'invention peut également contenir d'autres types de particules, par exemple, des particules d'oxyde de titane ou de zinc, d'aluminium.

Les tensioactifs convenant à la mise en oeuvre de la présente invention sont notamment les suivants :
(i) Tensioactif(s) anionique(s) :
   A titre d'exemple de tensioactifs anioniques utilisables, seuls ou mélanges, dans le cadre de la présente invention, on peut citer notamment les sels (en particulier, sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 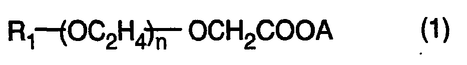 dans laquelle :
   R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
   A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

   Parmi tous ces tensioactifs anioniques, on préfère utiliser plus particulièrement les sels d'alkylsulfates et d'alkyléthersulfates, ainsi que leurs mélanges.
(ii) Tensioactif(s) amphotère(s) :
   Les agents tensioactifs amphotères peuvent être notamment des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
   Parmi les dérivés d'amines, on peut citer les produits commercialisés sous les dénomination MIRANOL, tels que décrits dans les brevets US-2 528 378 et US-2 781 354 et de structures :

   R₂-CONHCH₂CH₂-N(R₃)(R₄)(CH₂COO-) (2)

   dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
   et

   R₅-CONHCH₂CH₂-N(B)(C) (3)

   dans laquelle :
   B représente -CH₂CH₂OX', C représente -(CH₂)_{z}-Y', avec z =1 ou 2,
   X' désigne le groupement -CH₂CH₂-COOH ou un atome d'hydrogène
   Y' désigne -COOH ou le radical -CH₂-CHOH-SO3H
   R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

   Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Coco-amphodipropionic acid.
   A titre d'exemple on peut citer le cocoamphodiacetate commercialisé sous la dénomination commerciale MIRANOL C2M concentré par la société RHONE POULENC.
(iii) Tensioactif(s) non ionique(s) :
   Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'aminés tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

Avantageusement, les concentrations relatives des tensioactifs dans la composition sont celle-ci :
- les tensioactifs anioniques (i) sont présents à raison de 2 à 50 % en poids, de préférence de 3 à 20 % en poids, par rapport au poids total de la composition ;
- les tensioactifs amphotères (ii) sont présents à raison de 1 à 50 % en poids, de préférence de 1 à 20 % en poids, par rapport au poids total de la composition ;
- les tensioactifs non ioniques (iii) sont présents à raison de 1 à 50 % en poids, de préférence de 1 à 20 % en poids, par rapport au poids total de la composition.

On utilise de préférence un agent tensioactif anionique choisi parmi les alkyl(C₁₂₋C₁₄) sulfates de sodium, de triéthanolamine ou d'ammonium, les alkyl (C₁₂₋C₁₄)éthersulfates de sodium, de triéthanolamine ou d'ammonium oxyéthylénés à 2,2 moles d'oxyde d'éthylène, parmi les alkyl(C₁₂-C₁₄)amido sulfates de sodium, de triéthanolamine ou d'ammonium, le cocoyl iséthionate de sodium et l'alphaoléfine(C₁₄-C₁₆) sulfonate de sodium en mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylamidobétaïnes et les alkylbétaïnes en particulier la cocobétaïne commercialisée sous la dénomination "DEHYTON AB 30" en solution aqueuse à 32 % de MA par la société HENKEL.

### POLYMERE CATIONIOUE

Les polymères cationiques utilisés conformément à l'invention ont en général un poids moléculaire moyen en masse d'au moins 5000, préférentiellement d'au moins 10.000 et inférieur à 10.000.000 et plus particulièrement allant de 100.000 à 2.000.000. Ils ont en général des motifs contenant un atome d'azote tels que des motifs ammoniums quaternaires ou amino ou leurs mélanges. Leur densité de charge cationique est inférieure ou égale à 7 meq/g et, de préférence, supérieure ou égale à 0,05 meq/g, et plus préférentiellement, cette densité de charge est comprise entre 0,5 et 7 meq/g. La densité de charge peut être déterminée selon la méthode-Kjeldahl. Elle correspond en général à un pH de l'ordre de 3 à 9.

Parmi les polymères cationiques utilisables selon l'invention, on peut citer les copolymères de monomères vinyliques ayant des fonctions amines ou ammoniums quaternaires avec des monomères à insaturation éthylénique hydrosolubles tels que l'acrylamide, le méthacrylamide, les alkyl- ou dialkyl(méth)acrylamides, les alkyl(méth)-acrylates, le vinylcaprolactone, le vinylpyrrolidone ; ou biens d'autres monomères tels que les esters vinyliques, l'alcool vinylique, l'anhydride maléique, le propylène-glycol, l'éthylène glycol. Les groupes alkyls ou dialkyls des fonctions amines ou ammoniums sont de préférence en C₁-C₉ et plus préférentiellement en C₁-C₃.

Les amines peuvent être primaires, secondaires ou tertiaires. Les amines secondaires et tertiaires sont préférentielles.

Les monomères vinyliques amino-substitués peuvent être polymérisés sous leur forme amine puis éventuellement quaternisés. Les amines peuvent être également quaternisées après la formation du polymère. Par exemple, les fonctions aminés tertiaires peuvent être quaternisées par réaction avec un sel de formule R'X où R' est un radical alkyle de chaîne courte (en C₁-C₇ de préférence et plus particulièrement en C₁-C₃) et X est un anion formant un sel hydrosoluble avec l'ammonium quaternaire.

Parmi les monomères vinyliques à fonction amines ou ammoniums quaternaires, on peut citer par exemple des composés vinyliques substitués par un groupe du type dialkylaminoalkyl (méth)acrylate, monoalkyl-aminoalkyl (méth)acrylate ; des sels de trialkyl-méthacryloxyalkyl ammonium ; des sels diallyliques d'ammonium quaternaire ; des monomères vinyliques quaternaires ayant des cycles portant des atomes d'azote tels que pyridinium, imidazolium, pyrrolidone quaternisée comme alkylvinylimidazolium, alkyl-vinylpyridinium, les sels quaternaires d'alkylvinyl pyrrolidone. Les portions alkyls de ces monomères sont de préférence des alkyls en C₁-C₃ et plus préférentiellement des alkyls en C₁ ou C₂.

On peut également citer comme monomères vinyliques amino-substitués, les dialkylaminoalkyl (méth)acrylates, les dialkylaminoalkyl (méth)acrylamides. Les groupes alkyls ou dialkyls sont de préférence en C₁-C₉ et plus préférentiellement en C₁-C₃.

Les polymères cationiques de l'invention peuvent comprendre des mélanges de monomères vinyliques dérivés d'amines et/ou de monomères vinyliques dérivés d'ammoniums quaternaires et/ou d'autres monomères compatibles. On peut citer, à titre d'exemple :
- les copolymères de 1-vinylpyirolidone et de sel de 1-vinyl-3-méthylimidazolium (chlorure par exemple) (appelé Polyquaternium-16 dans le CTFA) tels que ceux vendus sous le nom LUVIQUAT par la société BASF ;
- les copolymères de 1-vinyl-2-pyrrolidone et de diméthylaminoéthylméthacrylate (appelés Polyquaternium-11 selon le CTFA) tels que ceux vendus sous le nom GAFQUAT (par exemple le GAFQUAT 755N) par la société GAF CORPORATION ;
- les homopolymères de chlorure de diméthyl-diallylammonium (Polyquaternium 5 selon le CTFA) et les copolymères d'acrylamide et de chlorure de diméthyl-diallylammonium (Polyquaternium-7 selon le CTFA) tels que ceux vendus sous le nom MERQUAT 550 et MERQUAT S par la Société MERCK ;
- les sels d'acides minéraux d'aminoalkylesters d'homo et de copolymères d'acides carboxyliques insaturés ayant de 3 à 5 atomes de carbone tels que ceux décrits dans le brevet USP 4 009 256.

Parmi les polymères cationiques utilisables, on peut citer aussi les polysaccharides cationiques tels que les dérivés de cellulose cationiques et les dérivés de l'amidon cationiques.

Parmi les polysaccharides cationiques, on peut citer les polymères de formule : 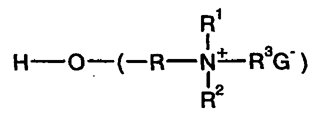 où:
H est un reste d'anhydroglucose tel que l'amidon ou un reste d'anhydroglucose cellulosique ;
R est un alkylène, un oxyalkylène, un polyoxyalkylène ou un hydroxyalkylène ou leurs mélanges ;
R¹, R² et R³, identiques ou différents, désignent un groupe alkyle, aryle, alkylaryle, arylalkyle, alkoxyalkyle ou alkoxyaryle ; chaque groupe contenant jusqu'à 18 atomes de carbone et le nombre total d'atomes de carbone par unité cationique est de préférence inférieure ou égale à 20.
G⁻ est un anion résultant de la quaternisation de l'amine NR¹R²R³.

Parmi les polymères cellulosiques cationiques, on peut citer ceux vendus par la société AMERCHOL CORP. sous les noms JR et LR tels que les sels d'hydroxyéthylcellulose quaternaires obtenus par réaction avec un époxyde susbtitué par un triméthylammonium (polyquaternium-10 selon le CTFA). On peut citer également les sels d'hydroxyéthyl-cellulose quaternaires obtenus par réaction avec un époxyde substitué par le lauryl-diméthylammonium (polyquaternium-24 selon le CTFA) comme ceux vendus sous le nom POLYMER LM200 par AMERCHOL CORP.

On peut citer également, comme polymères cationiques utilisables selon l'invention, les dérivés de gomme de guar cationiques tels que le chlorure d'hydroxypropyltrimonium de guar vendu sous les noms JAGUAR par la société CELANESE CORP.

On peut citer également les éthers celluloses quaternaires telles que celles décrites dans le brevet USP. 3 962 418 et les copolymères de cellulose étherifiés et d'amidon tels que ceux décrits dans le brevet US 3 958 581.

Les polymères cationiques sont présents, dans les compositions selon l'invention, dans des proportions allant, de préférence, de 0,01 à 5 % en poids et de préférence de 0,1 à 3 % en poids par rapport au poids total de la composition.

### COMPOSITION

Le milieu aqueux cosmétiquement acceptable peut être constitué uniquement par de l'eau ou par un mélange d'eau et d'un ou plusieurs solvants cosmétiquement acceptables ou par un ou plusieurs solvants cosmétiquement acceptables, tel qu'un alcool inférieur en C₁₋C₄, comme l'éthanol, l'isopropanol, le tertiobutanol, le n-butanol; les alkylèneglycols comme le propylèneglycol, les éthers de glycols.

De préférence, le milieu cosmétiquement acceptable contient de l'eau.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir, de préférence, jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement comprendre en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs cationiques, les polymères anioniques, non ioniques, amphotères ou cationiques autres que ceux de l'invention, les protéines, les hydrolysats de protéines, les hydroxyacides, les vitamines, les provitamines tels que le panthénol, les silicones volatiles ou non, linéaires ou cycliques, réticulées ou non, organomodifiées ou non.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas ou substantiellement pas, altérées par la ou les adjonctions envisagées.

Ces additifs sont, éventuellement, présents dans la composition selon l'invention dans des proportions pouvant aller de 0,0001 à 20% en poids par rapport au poids total de la composition. La quantité de chaque additif est déterminée facilement par l'homme du métier selon sa nature et sa fonction.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques, telles que notamment les cheveux, consistant à appliquer une composition telle que définie ci-dessus, puis à effectuer un rinçage à l'eau après un éventuel temps de pause.

Les compositions selon l'invention sont utilisées, de préférence, comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gel-douche pour le lavage et le conditionnement des cheveux et/ou de la peau, auquel cas ils sont appliqués sur la peau et/ou les cheveux humides et sont rincés après application.

Les compositions de l'invention peuvent également être utilisées en non rincé, et en particulier dans des lotions, dans des gels, dans des mousses ou dans dès aérosols.

Des exemples concrets, mais nullement limitatif, illustrant l'invention vont maintenant être donnés.

### EXEMPLES :

On réalise les deux shampooings ci-après :

**Shampooing A**

| | |
|---|---|
| Lauryl sulfate de triéthanolamine à 40 % m.a. | 31,3% |
| Cocoyl amidopropyl bétame (1) à 38 % m.a. | 6,6% |
| Polymère cationique (2) | 3,1 % |
| Carbonate de calcium en poudre (3) | 3 % |

| | |
|---|---|
| (1) en solution aqueuse (2) chlorure de diméthyl diallyl ammonium/acrylamide 50/50 en solution aqueuse à 8 % protégée (3) OMYA PUR 3S commercialisé par OMYA | |

Qsp pH=7
Qsp eau = 100

**Shampooing B**

| | |
|---|---|
| Lauryl sulfate de triéthanolamine à 40 % m.a. | 31,3 % |
| Alkyl (C1/C9) polyglucoside [1,4] (4) | 6,3 % |
| Polymère cationique (2) | 3,1 % |
| Carbonate de calcium en poudre (3) | 3 % |

| | |
|---|---|
| (2) chlorure de diméthyl diallyl ammonium/acrylamide 50/50 en solution aqueuse à 8 % protégée (3) OMYA PUR 3S commercialisé par OMYA (4) en solution aqueuse à 40 % | |

Qsp pH=7
Qsp eau = 100

La composition présente une texture agréable lors de l'application sur des cheveux humides. Sa rinçabilité est bonne. Les cheveux mouillés ne sont pas chargés et la mise en forme est aisée.

## Revendications

1. Composition cosmétique, notamment capillaire, comprenant, dans un milieu cosmétiquement acceptable :
(c) des particules solides contenant au moins 10 % en poids de carbonate de calcium ;
(d) une association d'au moins deux tensioactifs, choisie parmi les associations:
- d'au moins un tensioactif anionique (i) et d'au moins un tensioactif amphotère (ii) ou
- d'au moins un tensioactif anionique (i) et d'au moins un tensioactif non ionique (iii) ;
(c) un polymère cationique dont la densité de charge cationique est inférieure ou égale à 7 meq/g et, de préférence, supérieure ou égale à 0,05 meq/g.

2. Composition selon la revendication 1, **caractérisée par le fait que** les particules contenant au moins 10 % en poids de carbonate de calcium présentent une taille primaire moyenne en nombre comprise entre 2 nm et 2 µm, plus préférentiellement entre 5 et 500 nm, et plus préférentiellement encore entre 10 et 250 nm.

3. Composition selon la revendication selon l'une quelconque des revendications précédentes, **caractérisée par le fait que** les particules contiennent au moins 50 % en poids de carbonate de calcium, mieux encore au moins 70 % en poids, et encore mieux plus de 90 % en poids de carbonate de calcium.

4. Composition selon l'une quelconque des revendications précédentes, **caractérisée par le fait que** les particules sont des particules de carbonate de calcium substantiellement pur.

5. Composition selon l'une quelconque des revendications précédentes, **caractérisée par le fait que** les particules contenant du carbonate de calcium sont utilisées en une quantité comprise entre 0,01 % et 30 % en poids, et de préférence entre 0,05 % et 5 % en poids par rapport au poids total de la composition.

6. Composition selon l'une quelconque des revendications 1 à 5, **caractérisée par le fait que** le ou lesdits tensioactifs anioniques (i) sont présents à raison de 2 à 50 % en poids, de préférence de 3 à 20 % en poids, par rapport au poids total de la composition.

7. Composition selon l'une quelconque des revendications 1 à 6, **caractérisée par le fait que** le ou lesdits tensioactifs amphotères (ii) sont présents à raison de 1 à 50 % en poids, de préférence de 1 à 20 % en poids, par rapport au poids total de la composition.

8. Composition selon l'une quelconque des revendications 1 à 6, **caractérisée par le fait que** le ou lesdits tensioactifs non ioniques (iii) sont présents à raison de 1 à 50 % en poids, de préférence de 1 à 20 % en poids, par rapport au poids total de la composition.

9. Composition selon l'une quelconque des revendications précédentes, **caractérisée par le fait que** le rapport pondéral de concentrations du ou des tensioactifs anioniques aux tensioactifs non ioniques et/ou amphotères est supérieur à 1.

10. Composition selon l'une quelconque des revendications précédentes, **caractérisée par le fait que** ledit tensioactif anionique (i) est choisi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, parmi les alkyl(C₁₂-C₁₄)amido sulfates de sodium, de triédianolamine ou d'ammonium, le cocoyl iséthionate de sodium et l'alphaoléfine(C₁₄-C₁₆) sulfonate de sodium.

11. Composition selon l'une quelconque des revendications 1 à 7 et 9, **caractérisée par le fait que** ledit tensioactif amphotère (ii) est choisi parmi les dérivés d'aminés secondaires ou tertiaires aliphatiques, dans lesquels le radical aliphatique est une chaîne linéaire ou ramifiée comportant 8 à 22 atomes de carbone et contenant au moins un groupe anionique, les alkyl (C₈-C₂₆) bétaines, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

12. Composition selon l'une quelconque des revendications 1 à 6, 8 et 9, **caractérisée par le fait que** ledit tensioactif non ionique (iii) est choisi parmi les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

13. Composition selon l'une quelconque des revendications précédentes, **caractérisée par le fait que** la densité de charge cationique de polymère cationique (c) varie de 0,5 à 7 meq/g.

14. Composition selon l'une quelconque des revendications précédentes, **caractérisée par le fait que** le polymère cationique (c) est choisi dans le groupe constitué par :
a) les copolymères de monomères vinyliques ayant des fonctions amines primaires, secondaires, tertiaires ou ammoniums quaternaires
b) les polysaccharides cationiques dérivés de l'amidon ou dérivés de la cellulose ;
c) les polymères cationiques dérivés de la gomme de guar ;
d) les éthers celluloses quaternaires, les copolymères de cellulose éthérifiés et d'amidon.

15. Composition selon l'une quelconque des revendications précédentes, **caractérisée par le fait que** le polymère cationique (c) est présent dans des proportions allant de 0,01 à 5 % en poids et de préférence de 0,1 à 3 % en poids par rapport au poids total de la composition.

16. Composition selon l'une quelconque des revendications précédentes, **caractérisée par le fait qu'**elle comprend en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs cationiques, les polymères anioniques, non ioniques, amphotères ou cationiques autres que ceux de l'invention, les protéines, les hydrolysats de protéines, les hydroxyacides, les vitamines, les provitamines tels que le panthénol, les silicones volatiles ou non, linéaires ou cycliques, réticulées ou non, organomodifiées ou non.

17. Utilisation d'une composition telle définie dans l'une quelconque des revendications 1 à 16 comme shampooing.

18. Procédé de lavage et de conditionnement des cheveux consistant à appliquer une quantité efficace d'une composition telle que définie à l'une quelconque des revendications 1 à 16, puis à effectuer un rinçage à l'eau après un éventuel temps de pose.

## Claims

1. Cosmetic composition, especially a hair composition, comprising, in a cosmetically acceptable medium:
(a) solid particles containing at least 10% by weight of calcium carbonate;
(b) a combination of at least two surfactants, chosen from combinations:
- of at least one anionic surfactant (i) and of at least one amphoteric surfactant (ii), or
- of at least one anionic surfactant (i) and of at least one nonionic surfactant (iii);
(c) a cationic polymer whose cationic charge density is less than or equal to 7 meq/g and preferably greater than or equal to 0.05 meq/g.

2. Composition according to Claim 1, **characterized in that** the particles containing at least 10% by weight of calcium carbonate have a number-average primary size of between 2 nm and 2 µm, more preferably between 5 and 500 nm and even more preferably between 10 and 250 nm.

3. Composition according to either of the preceding claims, **characterized in that** the particles contain at least 50% by weight of calcium carbonate, better still at least 70% by weight and even better still more than 90% by weight of calcium carbonate.

4. Composition according to any one of the preceding claims, **characterized in that** the particles are particles of substantially pure calcium carbonate.

5. Composition according to any one of the preceding claims, **characterized in that** the particles containing calcium carbonate are used in an amount of between 0.01% and 30% by weight and preferably between 0.05% and 5% by weight relative to the total weight of the composition.

6. Composition according to any one of Claims 1 to 5, **characterized in that** said anionic surfactant(s) (i) is (are) present in a proportion of from 2% to 50% by weight and preferably from 3% to 20% by weight relative to the total weight of the composition.

7. Composition according to any one of Claims 1 to 6, **characterized in that** said amphoteric surfactant(s) (ii) is (are) present in a proportion of from 1% to 50% by weight and preferably from 1% to 20% by weight relative to the ' total weight of the composition.

8. Composition according to any one of Claims 1 to 6, **characterized in that** said nonionic surfactant(s) (iii) is (are) present in a proportion of from 1% to 50% by weight and preferably from 1% to 20% by weight relative to the total weight of the composition.

9. Composition according to any one of the preceding claims, **characterized in that** the concentration weight ratio of the anionic surfactant(s) to the nonionic and/or amphoteric surfactants is greater than 1.

10. Composition according to any one of the preceding claims, **characterized in that** said anionic surfactant (i) is chosen from sodium, triethanolamine or ammonium (C₁₂-C₁₄) alkyl sulfates, sodium, triethanolamine or ammonium (C₁₂-C₁₄) alkyl ether sulfates oxyethylenated with 2.2 mol of ethylene oxide, sodium, triethanolamine or ammonium (C₁₂-C₁₄) alkylamido sulfates, sodium cocoyl isethionate and sodium α-(C₁₄-C₁₆) olefin sulfonate.

11. Composition according to any one of Claims 1 to 7 and 9, **characterized in that** said amphoteric surfactant (ii) is chosen from aliphatic secondary or tertiary amine derivatives in which the aliphatic radical is a linear or branched chain containing 8 to 22 carbon atoms and containing at least one anionic group, (C₈-C₂₀) alkylbetaines, sulfobetaines, (C₈-C₂₀) alkylamido (C₁-C₆) alkylbetaines or (C₈-C₂₀) alkylamido (C₁-C₆) alkylsulfobetaines.

12. Composition according to any one of Claims 1 to 6, 8 and 9, **characterized in that** said nonionic surfactant (iii) is chosen from polyethoxylated, polypropoxylated or polyglycerolated fatty acids, alkylphenols, α-diols or alcohols having a fatty chain containing, for example, 8 to 18 carbon atoms, it being possible for the number of ethylene oxide or propylene oxide groups to range in particular from 2 to 50 and for the number of glycerol groups to range in particular from 2 to 30.

13. Composition according to any one of the preceding claims, **characterized in that** the cationic charge density of the cationic polymer (c) ranges from 0.5 to 7 meq/g.

14. Composition according to any one of the preceding claims, **characterized in that** the cationic polymer (c) is chosen from the group consisting of:
a) copolymers of vinyl monomers containing primary, secondary or tertiary amine functions or quaternary ammonium functions;
b) cationic polysaccharides derived from starch or derived from cellulose;
c) cationic polymers derived from guar gum;
d) quaternary cellulose ethers, and copolymers of etherified cellulose and of starch.

15. Composition according to any one of the preceding claims, **characterized in that** the cationic polymer (c) is present in proportions ranging from 0.01% to 5% by weight and preferably from 0.1% to 3% by weight relative to the total weight of the composition.

16. Composition according to any one of the preceding claims, **characterized in that** it also comprises at least one additive chosen from foam synergists such as C₁₀-C₁₈ 1,2-alkanediols or fatty alkanolamides derived from monoethanolamine or from diethanolamine, silicone or nonsilicone sunscreens, cationic surfactants, anionic, nonionic, amphoteric or cationic polymers other than those of the invention, proteins, protein hydrolysates, hydroxy acids, vitamins, provitamins such as panthenol, and volatile or nonvolatile, linear or cyclic, crosslinked or noncrosslinked, organomodified or non-organomodified silicones.

17. The use of a composition as defined in any one of Claims 1 to 16, as a shampoo.

18. A process for washing and conditioning the hair, which consists in applying an effective amount of a composition as defined in any one of Claims 1 to 16, and then in rinsing with water after an optional leave-in time.

## Patentansprüche

1. Kosmetische Zusammensetzung, insbesondere für die Haarbehandlung, die in einem kosmetisch akzeptablen Medium enthält:
(a) feste Partikel, die mindestens 10 Gew.-% Calciumcarbonat umfassen;
(b) eine Kombination aus mindestens zwei grenzflächenaktiven Stoffen, die unter den folgenden Kombinationen gewählt ist:
- mindestens ein anionischer grenzflächenaktiver Stoff (i) und mindestens ein amphoterer grenzflächenaktiver Stoff (ii) oder
- mindestens ein anionischer grenzflächenaktiver Stoff (i) und mindestens ein nichtionischer grenzflächenaktiver Stoff (iii);
(c) ein kationisches Polymer, bei dem die Dichte der kationischen Ladung höchstens 7 meq/g und vorzugsweise mindestens 0,05 meq/g beträgt.

2. Zusammensetzung nach Anspruch 1, **dadurch gekennzeichnet, dass** die Partikel, die mindestens 10 Qew.% Calciumcarbonat umfassen, eine zahlenmittlere Primärgröße von 2 nm bis 2 µm, vorzugsweise 5 bis 500 nm und noch bevorzugter 10 bis 250 nm aufweisen.

3. Zusammensetzung nach einem der vorhergehenden Ansprüche, **dadurch gekennzeichnet, dass** die Partikel mindestens 50 Gew.-% Calciumcarbonat, besser mindestens 70 Gew.-% und noch besser mindestens 90 Gew.-% Calciumcarbonat enthalten.

4. Zusammensetzung nach einem der vorhergehenden Ansprüche, **dadurch gekennzeichnet, dass** die Partikel im Wesentlichen reine Calciumcarbonatpartikel sind.

5. Zusammensetzung nach einem der vorhergehenden Ansprüche, **dadurch gekennzeichnet, dass** die Partikel, die Calciumcarbonat enthalten, in einer Menge von 0,01 bis 30 Gew.-% und vorzugsweise 0,05 bis 5 Gew.-%, bezogen auf das Gesamtgewicht der Zusammensetzung, verwendet werden.

6. Zusammensetzung nach einem der Ansprüche 1 bis 5, **dadurch gekennzeichnet, dass** der oder die anionische(n) grenzflächenaktive(n) Stoff(e) (i) in einer Menge von 2 bis 50 Gew.-% und vorzugsweise 3 bis 20 Gew.-%, bezogen auf das Gesamtgewicht der Zusammensetzung, enthalten sind.

7. Zusammensetzung nach einem der Ansprüche 1 bis 6, **dadurch gekennzeichnet, dass** der oder die amphotere(n) grenzflächenaktive(n) Stoff(e) (ii) in einer Menge von 1 bis 50 Gew.-% und vorzugsweise 1 bis 20 Gew.-%, bezogen auf das Gesamtgewicht der Zusammensetzung, enthalten sind.

8. Zusammensetzung nach einem der Ansprüche 1 bis 6, **dadurch gekennzeichnet, dass** der oder die nichtionische(n) grenzflächenaktive(n) Stoff(e) (iii) in einer Menge von 1 bis 50 Gew.-% und vorzugsweise 1 bis 20 Gew.-%, bezogen auf das Gesamtgewicht der Zusammensetzung, enthalten sind.

9. Zusammensetzung nach einem der vorhergehenden Ansprüche, **dadurch gekennzeichnet, dass** das Gewichtsverhältnis der Konzentrationen des oder der anionischen grenzflächenaktiven Stoffe und der nichtionischen und/oder amphoteren grenzflächenaktiven Stoffe über 1 liegt.

10. Zusammensetzung nach einem der vorhergehenden Ansprüche, **dadurch gekennzeichnet, dass** der anionische grenzflächenaktive Stoff (i) unter Natriumalkyl(C₁₂₋₁₄)sulfaten, Triethanolaminalkyl(C₁₂₋₁₄)sulfaten oder Ammoniumalkyl(C₁₂₋₁₄)sulfaten, Natriumalkyl(C₁₂₋₁₄)ethersulfaten, Triethanolaminalkyl(C₁₂₋₁₄)ethersulfaten oder Ammoniumalkyl(C₁₂₋₁₄)ethersulfaten, die mit 2,2 mol Ethylenoxid ethoxyliert sind, Natriumalkyl(C₁₂₋₁₄)amidosulfaten, Triethanolaminalkyl(C₁₂₋₁₄) amidosulfaten oder Ammoniumalkyl(C₁₂₋₁₄)amidosulfaten, Natriumcocoylisethionat und Natrium-α-olefin(C₁₄₋₁₆)sulfonat ausgewählt ist.

11. Zusammensetzung nach einem der Ansprüche 1 bis 7 und 9, **dadurch gekennzeichnet, dass** der amphotere grenzflächenaktive Stoff (ii) unter den aliphatischen sekundären oder tertiären Aminderivaten, bei denen die aliphatische Gruppe eine geradkettige oder verzweigte Gruppe mit 8 bis 22 Kohlenstoffatomen ist und mindestens eine anionische Gruppe aufweist, Alkyl(C₈₋₂₀)betainen, Sulfobetainen, Alkyl(C₈₋₂₀)amidoalkyl(C₁₋₆)betainen oder Alkyl(C₈₋₂₀)amidoalkyl(C₁₋₆)sulfobetainen ausgewählt ist.

12. Zusammensetzung nach einem der Ansprüche 1 bis 6, 8 und 9, **dadurch gekennzeichnet, dass** der nichtionische grenzflächenaktive Stoff (iii) unter den Alkoholen, α-Diolen, Alkylphenolen oder Fettsäuren, die polyethoxyliert, polypropoxyliert oder mehrfach mit Glycerin verethert sind und eine Fettkette mit beispielsweise 8 bis 18 Kohlenstoffatomen aufweisen, wobei die Anzahl der Ethylenoxidgruppen oder Propylenoxidgruppen insbesondere im Bereich von 2 bis 50 und die Anzahl der Glyceringruppen insbesondere im Bereich von 2 bis 30 liegen kann, ausgewählt ist.

13. Zusammensetzung nach einem der vorhergehenden Ansprüche, **dadurch gekennzeichnet, dass** die Dichte der kationischen Ladung des kationischen Polymers (c) im Bereich von 0, 5 bis 7 meq/g liegt.

14. Zusammensetzung nach einem der vorhergehenden Ansprüche, **dadurch gekennzeichnet, dass** das kationische Polymer (c) unter den folgenden Verbindungen ausgewählt ist:
a) Copolymeren von Vinylmonomeren mit primären, sekundären, tertiären Aminfunktionen oder quartären Ammoniumfunktionen
b) kationischen Polysacchariden, die von Stärke abgeleitet sind oder die von Cellulose abgeleitet sind;
c) kationischen Polymeren, die von Guargummi abgeleitet sind;
d) quartären Celluloseethern, veretherten Cellulose-Stärke-Copolymercn.

15. Zusammensetzung nach einem der vorhergehenden Ansprüche, **dadurch gekennzeichnet, dass** das kationische Polymer (c) in einer Menge von 0,01 bis 5 Gew.-% und vorzugsweise 0,1 bis 3 Gew.%, bezogen auf das Gesamtgewicht der Zusammensetzung, vorliegt.

16. Zusammensetzung nach einem der vorhergehenden Ansprüche, **dadurch gekennzeichnet, dass** sie ferner mindestens einen Zusatzstoff enthält, der unter den Schaumsynergisten, wie (C₁₀₋₁₈)-1,2-Alkandiolen oder von Mono- oder Diethanolamin abgeleiteten Alkanolfettamiden, siliconierten oder nicht siliconierten Sonnenschutzfiltern, kationischen grenzflächenaktiven Stoffen, anionischen, nichtionischen, amphoteren oder kationischen Polymeren, die von den erfindungsgemäßen verschieden sind, Proteinen, Proteinhydrolysaten, Hydroxysäuren, Vitaminen, Provitaminen, wie Panthenol, flüchtigen oder nicht flüchtigen, geradkettigen oder cyclischen, vernetzten oder nicht vernetzten, organomodifizierten oder nicht organomodifizierten Siliconen ausgewählt ist.

17. Verwendung einer Zusammensetzung nach einem der Ansprüche 1 bis 16 als Haarwaschmittel.

18. Verfahren zur Reinigung und Konditionierung der Haare, das darin besteht, eine wirksame Menge einer Zusammensetzung nach einem der Ansprüche 1 bis 16 aufzutragen und anschließend nach einer gegebenenfalls abgewarteten Einwirkzeit mit Wasser zu spülen.
